# EUROPEAN PATENT APPLICATION

(11) **EP 4 732 801 A2**
(43) Date of publication of application: **29.04.2026**
(21) Application number: 26157736.5
(22) Date of filing: 18.05.2023
(51) Int. Cl.: A61B 18/18

(54) **A LIGHT TREATMENT DEVICE**

(30) Priority: 02.06.2022 EP 22176914
(62) Divisional of application: 23727558.1
(71) Applicant: Koninklijke Philips N.V., 5656 AG Eindhoven (NL)
(72) Inventor: VAN ABEELEN, Frank Anton, 5656AE Eindhoven (NL); VERHAGEN, Rieko, Eindhoven (NL); BOAMFA, Marius Iosif, Eindhoven (NL); THUMMA, Kiran Kumar, Eindhoven (NL); NUIJS, Antonius Maarten, Eindhoven (NL)
(74) Representative: Philips Intellectual Property & Standards

(57) **Abstract**

According to an aspect, there is provided a treatment device (2; 30; 60; 130) for performing light-based treatment operations on or to a subject. The treatment device (2; 30; 60; 130) comprises a light source (12) for generating light for performing the treatment operation; a dichroic filter (34; 64; 104; 134) arranged at a first angle with respect to the incident light such that the incident light is separated into a transmitted light component and a reflected light component according to a cut-off wavelength of the dichroic filter (34; 64; 104; 134), wherein the transmitted light component is transmitted through the dichroic filter (34; 64; 104; 134) and the reflected light component is reflected by the dichroic filter (34; 64; 104; 134); a light exit window (10) through which a light component is emitted from the treatment device (2; 30; 60; 130); a beam dump (44; 74; 140) configured to absorb a light component; and a heat sink (46; 76; 142) coupled to the beam dump (44; 74; 140) to dissipate heat from the beam dump (44; 74; 140). The light exit window (10) and beam dump (44; 74; 140) are arranged with respect to the dichroic filter (34; 64; 104; 134) such that one of the transmitted light component and the reflected light component is emitted from the treatment device (2; 30; 60; 130) via the light exit window (10) and the other one of the transmitted light component and reflected light component is incident on the beam dump (44; 74; 140). The dichroic filter (34; 64; 104; 134) is provided on a first surface of a solid dichroic prism (38; 68) or an internal surface in a solid dichroic cuboid.

## Description

### FIELD OF THE INVENTION

This disclosure relates to a treatment device for performing light-based treatment operations on or to a subject.

### BACKGROUND OF THE INVENTION

Techniques for the removal of unwanted hairs include shaving, electrolysis, plucking, laser and light therapies (known as photoepilation) and injection of therapeutic anti-androgens. Light-based technologies are also used in other types of dermatological treatments, including skin rejuvenation and treating acne.

Light-based hair treatments inhibit the growth of hair by exposing the skin to bright flashes or pulses of light (in case of non-coherent sources), known as intense pulsed light (IPL). Through the use of an appropriate configuration of the light energy, i.e. in terms of wavelength, intensity and/or pulse duration (if the light is to be pulsed), selective heating of the hair root and subsequent temporary or permanent damage to the hair follicle can be achieved. The IPL may be generated by a high intensity light source such as a gas discharge lamp (e.g., a Xenon flash lamp). The light penetrates the skin and is absorbed, among other places, in the root of the hair by the pigment melanin. This causes an increase in the temperature of the root of the hair and subsequently the temperature of the surrounding tissue. The generated heat damages the hair follicles, and the growth of the hair is inhibited if the temperature rise is sufficient. This process is known as photothermolysis. When the treatment is repeated in intervals of 2 to 4 weeks, a long-lasting hair reduction is achieved.

The main optical components of the light arrangement in a typical IPL device are a light source such as a flash lamp, a concave back reflector, side reflectors and an absorption filter. The flash lamp emits light in all directions. The back reflector and side reflectors form a reflective cavity around the lamp that directs the light towards the skin (i.e. the reflective cavity can collimate the light emitted by the lamp). A Xenon flash lamp can be used. As these lamps have a broad emission spectrum, the IPL device can comprise a long-pass absorption filter with a cut-off between 500 and 600 nanometres (nm) to prevent shorter wavelengths of light from reaching the skin. These shorter wavelengths are typically absorbed by haemoglobin in the blood and would otherwise cause discomfort and side effects to the subject, whereas the longer wavelengths of light are passed by the filter and are incident on the subject to perform the photothermolysis process on the skin/hairs.

Due to the absorption of the unwanted light (the shorter wavelengths of light), the absorption filter gets hot. When flashing an IPL device for a prolonged period at high repetition rate, the absorption filter gradually increases in temperature and may reach temperatures in excess of 200°C. This high temperature is perceived as unpleasant by the subject or device user through the radiative heat passing through the aperture (light exit window) to the skin as well as from the aperture materials themselves becoming too hot to touch. In addition, the filter may exceed safe touching temperatures. This may be a problem because the filter can be exposed to the user when an attachment is exchanged or cleaned. Furthermore, the cut-off wavelength may shift as a function of the filter temperature.

As an alternative to the use of an absorption filter to separate the light into the treatment light (i.e. the light that is to effect the hair removal operation) and the unwanted light (e.g. shorter wavelengths of light), the absorption filter may be replaced by a dichroic filter, such as a long-pass dichroic reflectance filter or a short-pass dichroic filter reflectance filter. However, in this case the unwanted light is still present in the treatment device and can 'reflect around' the interior of the treatment device, potentially damaging components, and also exit the treatment device through the light exit window. In these arrangements, while the dichroic filter acts as the primary separator for the generated light into the treatment light and the unwanted light, an absorption filter can be provided at or near the light exit window to absorb any 'stray' unwanted light that would otherwise exit the treatment device. However, where much of the unwanted light separated by the dichroic filter reflects around the interior of the treatment device, the absorption filter will absorb a lot of light, leading to the heating problems outlined above.

For example, a large fraction of the light reflected by the dichroic filter can find its way back to the absorption filter via multiple reflections in the reflective cavity surrounding the lamp, until it is incident on the dichroic filter at a large angle. At large angles, the dichroic filter will inevitably have a leak and transmit that component of light to the absorption filter. Through this recycling process, a significant part of the energy at the shorter wavelength end of the spectrum will still end up being absorbed in the absorption filter, which will still get hot. In addition, some energy is transferred to the lamp, and that is not desirable because it can have a negative impact on the lifetime of the lamp.

Therefore further solutions for the light arrangement of treatment devices are desirable to address the heating issue.

US5782895A discloses an illuminator for photodynamic therapy which includes a bulb, a heat dissipator and a filter assembly. The filter assembly includes the following components in an optical path - a high-pass filter to filter out light having wavelengths below a first wavelength, a low-pass dichroic filter to filter out light having wavelengths above a second wavelength and a dichroic mirror which reflects having wavelengths between the first and second wavelength. Light transmitted through the dichroic mirror is directed towards the heat dissipator.

In the lighting system of US2011/0051216A1, light generated by a light source is focused and collimated to a dichroic beam splitter which transmits the light to the subject and reflects unwanted energy towards heat trap 48 for heat dissipation.

US6413268B1 discloses a UV phototherapy apparatus containing a UV arc lamp whose light is targeted to a treatment handpiece or a fan and heat sink assembly via dichroic mirrors.

US4048490A discloses a dichroic filter system for delivering UV light, where the UV light is directed to the workpiece and undesired IR light is absorbed by a dichroic filter and absorber arrangement. The absorber further comprises a heat dissipator.

### SUMMARY OF THE INVENTION

According to a first specific aspect, there is provided a treatment device for performing a light-based treatment operation on or to a subject. The treatment device comprising a light source for generating light for performing the treatment operation; a dichroic filter arranged at a first angle with respect to incident light such that the incident light is separated into a transmitted light component and a reflected light component according to a cut-off wavelength of the dichroic filter, wherein the transmitted light component is transmitted through the dichroic filter and the reflected light component is reflected by the dichroic filter; a light exit window arranged with respect to the dichroic filter such that one of the transmitted light component and the reflected light component is emitted from the treatment device via the light exit window; a beam dump configured and arranged with respect to the dichroic filter such that the other one of the transmitted light component and reflected light component is incident on the beam dump and absorbed by the beam dump; and a heat sink coupled to the beam dump to dissipate heat from the beam dump. In the first aspect, the dichroic filter is provided on a surface of a solid dichroic prism or that in a solid dichroic cuboid. The solid guides the incident light from light source to the subject and the dichroic separation of the incident beam takes place within this solid medium. With solid light guides, light that is injected within the correct range of angles becomes trapped inside the guide because of total internal reflection. Once trapped, the light remains inside the guide until for example it encounters an interface at less than the TIR critical angle. Thus, the first aspect provides that the unwanted light component is directed to the beam dump where it is absorbed, and the heat generated in the beam dump by the absorption of light is dissipated via the coupled heat sink. This reduces the build-up of heat in the treatment device, and thereby improves the user experience when handling the treatment device. At the same time, the combination of the solid medium with the dichroic filter provides a more effective confinement of treatment light (i.e. minimising path losses) while transmitting desired wavelengths to the subject.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is an illustration of an exemplary treatment device;
Fig. 2 is an illustration of part of a treatment device according to a first set of embodiments;
Fig. 3 is an illustration of part of a treatment device according to a second set of embodiments;
Figs. 4(a) and (b) show some exemplary dichroic filter and prism configurations;
Fig. 5 is an illustration of part of a treatment device according to a third set of embodiments; and
Fig. 6 is an illustration of a part of a treatment device according to a fourth set of embodiments.

### DETAILED DESCRIPTION OF THE EMBODIMENTS

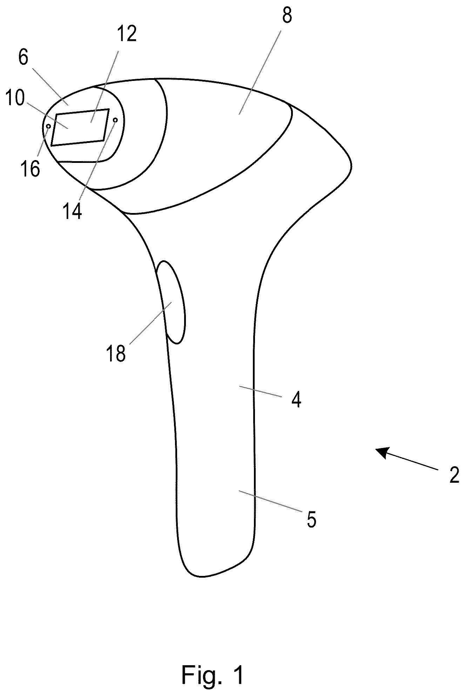

Fig. 1 is an illustration of an exemplary treatment device 2 that can be used to apply a light treatment to an area of skin. It will be appreciated that the treatment device 2 in Fig. 1 is merely presented as an example of a hand-held treatment device 2 that the invention can be used with, and the treatment device 2 is not limited to the form shown in Fig. 1 or to being a hand-held treatment device. The treatment device 2 is for use on a body of a subject (e.g. a person or an animal), and is to be held in one or both hands of a user during use. The treatment device 2 is to perform some treatment operations to hairs and/or skin of the body of the subject using light when the treatment device 2 is close to or in contact with a body part of the subject. The treatment operations include the removal of hairs by light therapies (known as a photoepilation treatment or Intense Pulsed Light treatment). The treatment operations can also include skin rejuvenation. Such treatments can be performed using pulses of light, or by applying light continuously or for longer periods of time.

As described herein, the treatment device 2 is operated or used by a 'user', and the treatment device 2 is used on a body of a 'subject'. In some cases, the user and the subject is the same person, i.e. the treatment device 2 is held in a hand and used by a user on themselves (e.g. used on the skin on their leg). In other cases, the user and the subject are different people, e.g. the treatment device 2 is held in a hand and used by a user on someone else.

The exemplary treatment device 2 comprises a housing 4 that includes at least a handle portion 5 and a head portion 6. The handle portion 5 is shaped to enable the user to hold the treatment device 2 with one hand. The head portion 6 is at a head end 8 of the housing 4, and the head portion 6 is to be placed into contact with the subject in order for the treatment operation to be performed on the body or skin of the subject at the position that the head portion 6 is in contact with the body or skin.

The treatment device 2 is for performing a treatment operation using light, such as light pulses. Thus, in Fig. 1 the head portion 6 comprises a light exit window 10, also referred to as an aperture or treatment window, that is arranged in or on the housing 4 so that the light exit window 10 can be placed adjacent to or on (e.g. in contact with) the skin of the subject. The treatment device 2 includes a light source 12 (not directly visible in Fig. 1) that is for generating light that is to be applied to the skin of the subject via the light exit window 10 and effect a treatment operation. The light source 12 is arranged in the housing 4 so that the light pulses are provided from the light source 12 through the light exit window 10. The aperture/light exit window 10 may be in the form of an opening at the head end 8 of the housing 4, or it may be in the form of a window (including a waveguide) that is transparent or semitransparent to the light (i.e. the light can pass through the window).

In the exemplary embodiment shown in Fig. 1, the light exit window 10 has a generally rectangular shape, which results in a generally rectangular-shaped skin treatment region (also referred to herein as a treatment area) on the skin. It will be appreciated that the light exit window 10 can have any other desired shape. For example, the light exit window 10 can be square, elliptical, circular, or any other polygonal shape.

The light source 12 can generate light pulses of any suitable or desired wavelength (or range of wavelengths) and/or intensities. For example, the light source 12 can generate visible light, infrared (IR) light and/or ultraviolet (UV) light. The light source 12 can comprise any suitable type of light source, such as a gas discharge lamp, one or more light emitting diodes (LEDs), a laser or lasers, etc.

The light source may be a gas discharge lamp. The gas discharge lamp may comprise a gas in a housing (e.g. a tube), where the gas is typically a noble gas, such as Xenon, Argon or Krypton, or a mixture of such gases. The gas discharge lamp may be a flash lamp, e.g., a Xenon flash lamp.

The light source 12 can provide light pulses with spectral content in the 560-1200 nanometre (nm) range for a duration of around 2 milliseconds (ms), as these wavelengths heat melanin in the hair and hair root by absorption, which puts the hair follicles in a resting phase, preventing hair regrowth, while absorption by other chromophores in the skin (e.g. haemoglobin, water) is limited.

The light source 12 can be configured to provide pulses of light or continuous light. That is, the light source 12 can be configured to generate light at a high intensity for a short duration (e.g. less than 1 second). The intensity of the light should be high enough to effect the treatment operation on the skin or body part adjacent the light exit window 10.

The illustrated treatment device 2 also includes two skin contact sensors 14, 16 positioned on or in the head portion 6 that are used to determine whether the head portion 6 is in contact with the skin before the light is generated to avoid the light being directed into the eyes of the user or subject.

The illustrated treatment device 2 also includes a user control 18 that can be operated by the user to activate the treatment device 2 so that the head portion 6 performs the required treatment operation on the body of the subject (e.g. the generation of one or more light pulses by the light source 12). The user control 18 may be in the form of a switch, a button, a touch pad, etc. A user control 18 may also be used to switch between different treatment operations, or different settings for those treatment operations.

As noted above, a dichroic filter can be used in a light arrangement of a treatment device to separate the relatively-broad spectrum light generated by the light source into the treatment light (i.e. the light that is to be used to effect the hair removal operation or other skin/hair treatment) and the unwanted light (i.e. that is not suitable for effecting the treatment operation and/or is detrimental to the subject). However, it is still necessary to deal with the unwanted light that is still present in the treatment device.

According to the techniques described herein, a beam dump is provided in the treatment device to absorb the unwanted light, and a heat sink is provided that is coupled to the beam dump to dissipate heat from the beam dump.

According to particular embodiments, a treatment device is provided that is for performing light-based treatment operations on or to a subject, such as hair removal, photoepilation. The treatment device comprises a light source for generating light for performing the treatment operation, a dichroic filter arranged at a first angle with respect to the light incident from the light source such that the incident light is separated into a transmitted light component and a reflected light component according to a cut-off wavelength of the dichroic filter (noting that in practice the cut-off wavelength is not typically a sharp cut-off, but has a transition across a finite wavelength width). The transmitted light component is the component of the generated light that is transmitted through the dichroic filter and the reflected light component is the component of the generated light that is reflected by the dichroic filter. The treatment device comprises a light exit window through which one of the light components is emitted from the treatment device to perform the treatment operation on the subject. The light exit window and beam dump are arranged with respect to the dichroic filter such that one of the transmitted light component and the reflected light component is emitted from the treatment device via the light exit window, and the other one of the transmitted light component and reflected light component is incident on the beam dump. In other words, one of the transmitted light component and the reflected light component is emited from the treatment device via the light exit window. The other one of the transmitted light component and reflected light component is incident on the beam dump and absorbed by the beam dump. Thus, in both configurations the unwanted light component is directed to the beam dump where it is absorbed, and the heat generated in the beam dump by the absorption of light is dissipated via the coupled heat sink. This reduces the build-up of heat in the treatment device, and thereby improves the user experience when handling the treatment device.

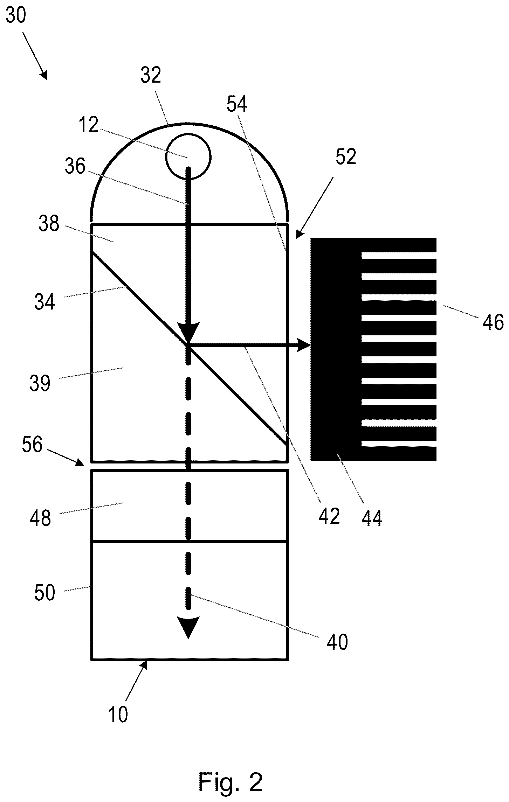

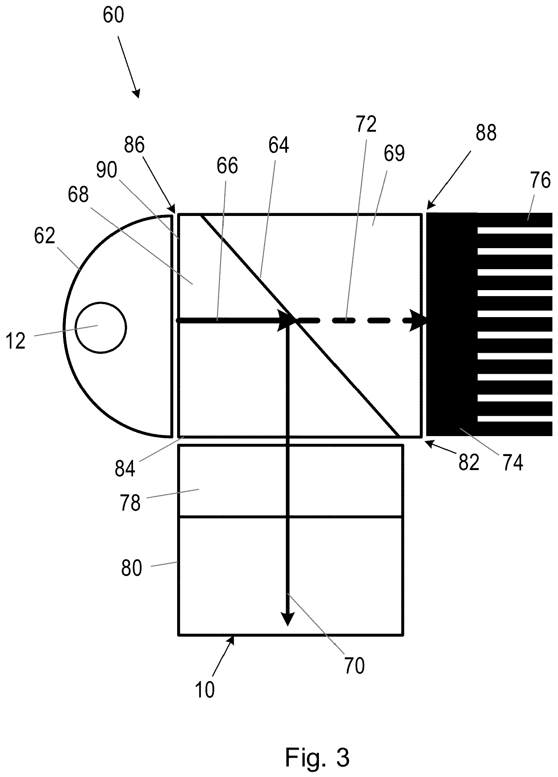

Two alternative configurations of the dichroic filter, light exit window and beam dump are primarily envisaged herein. In a first configuration, which is illustrated with respect to Fig. 2, the dichroic filter is such that the transmitted light component is the component of the light that is to perform the treatment operation, and the transmitted light component exits the treatment device via the light exit window. The reflected light component is incident on, and is received by, the beam dump. In a second configuration, which is illustrated with respect to Fig. 3, the dichroic filter is such that the reflected light component is the component of the light that is to perform the treatment operation, and the reflected light component exits the treatment device via the light exit window. The transmitted light component is incident on, and is received by, the beam dump.

As used herein, the term "treatment light" refers to the light component of the generated light that is to be used to perform the treatment operation, and is the light component that is intended to exit the treatment device via the light exit window. The term "unwanted light" refers to the light component of the generated light that is not to be used to perform the treatment operation (for example because the light doesn't have a suitable wavelength and/or that the light would cause undesirable effects on the subject), and is the light component that is intended to be absorbed by the beam dump.

In some embodiments, which are also illustrated in Figs. 2 and 3, in addition to the dichroic filter, the treatment device can also comprise an absorption filter that is arranged with respect to the dichroic filter and the light exit window such that the treatment light that is emitted from the treatment device via the light exit window passes through the absorption filter. The absorption filter is configured to absorb the unwanted light (i.e. the absorption filter is configured to absorb light having wavelengths corresponding to the unwanted light), in case any of the unwanted light finds its way to the light exit window.

Thus, Fig. 2 is an illustration of part of a treatment device 30 according to a first set of embodiments. The treatment device 30 comprises a light source 12 arranged in a cavity housing 32 of the treatment device 30. The light source 12 can be controllable or configured to generate pulses of light (e.g. IPL), or continuous, or relatively continuous, light.

The cavity housing 32 forms a reflective cavity for the light source 12 so that generated light is directed generally towards a dichroic filter 34, as indicated by arrow 36. The dichroic filter 34 is part of a prism 38. The dichroic filter 34 may be a thin-film dichroic filter. The prism 38 is located at the exit of the reflective cavity formed by the cavity housing 32. The dichroic filter 34 may be formed on one of the surfaces of the prism 38, in which case a second prism 39 is provided that abuts the surface with the dichroic filter 34 in the prism 38. Alternatively, the dichroic filter 34 may be formed on one of the surfaces of prism 39, in which case the first prism 38 abuts the surface with the dichroic filter in prism 39. The light transmitted through the dichroic filter 34 passes through the second prism 39. Alternatively, the dichroic filter 34 may be an internal surface of a generally cubic or cuboid prism (and so comprising prism elements 38 and 39 in Fig. 2).

The prism 38 can be a solid prism, in which case the second prism 39 may also be a solid prism.

The dichroic filter 34 is arranged at a first angle with respect to the incident light (arrow 36), and acts to filter the incident light 36 by transmitting some of the light in the incident light 36 through the dichroic filter 34 and reflecting some of the light in the incident light 36 from the dichroic filter 34 according to a cut-off wavelength of the dichroic filter.

In the embodiment illustrated in Fig. 2, the light that is to be the treatment light is light having wavelengths above the cut-off wavelength, and thus the dichroic filter 34 is configured to transmit light having wavelengths above the cut-off wavelength, with light having wavelengths below the cut-off wavelength being reflected by the dichroic filter 34. The transmitted light (the treatment light, in this embodiment) is indicated by arrow 40, and the reflected light (the unwanted light, in this embodiment) is indicated by arrow 42. The cut-off wavelength can be between 500 nm and 600 nm, e.g. 530 nm or 565 nm. If the treatment is other than hair removal/reduction, the cut-off wavelength may have a different value. The cut-off wavelength is defined herein at the average angle of incidence of the incident light on the dichroic filter.

The transmitted light 40 is directed towards the light exit window 10, through which it can exit the treatment device 30. The reflected light 42 is directed towards a beam dump 44 that absorbs light, and in particular absorbs light having wavelengths at least in the range corresponding to the wavelengths of light in the reflected light 42. Coupled to the beam dump 44 is a heat sink 46 that acts to dissipate heat from the beam dump 44.

In this illustrated embodiment, the dichroic filter 34 is arranged at an angle of 45° with respect to the incident light 36, with the beam dump 44/heat sink 46 arranged at an angle of 90° with respect to the incident light 36. However, in alternative embodiments, the angle of the dichroic filter 34 with respect to the incident light 36 can be other than 45°. For example, the angle may be close to 45°, or any angle between 30° and 60°. Likewise, the angle of the beam dump 44 with respect to the incident light 36 may be other than 90°, although preferably the beam dump 44 is positioned with respect dichroic filter 34 to capture as much of the reflected light component 42 as possible.

As the beam dump 44 and heat sink 46 are positioned away from the light exit window 10, which is typically placed into contact with the skin of the subject, the treatment device 30 will not feel as hot to the subject during use. Moreover, the absorbed heat is dissipated efficiently so that the temperature of the heat sink is typically lower than the temperatures in excess of 200 °C found in the absorption filter of a conventional design.

The beam dump 44 can be formed from any suitable material and have any suitable construction. For example, the beam dump can be a conical beam trap of a blackened material, such as a metal. More simply, the beam dump 44 can be a "black" layer with high absorption and low reflectance. Specific examples of suitable black materials are black anodized aluminium, nickel-phosphorus alloy and coatings based on carbon nanotubes.

The heat sink 46 can be formed from any suitable material and have any suitable construction. For example, the heat sink can be a base with fins to increase the contact area with an air flow through the fins. It can be made out of a single piece of material like copper or an aluminium alloy. Alternatively, sheet-metal fins can be soldered onto the base.

The beam dump and the heat sink may be combined into a single element, e.g. made of anodized aluminium.

As noted above, in the embodiment of Fig. 2, in addition to the dichroic filter 34, the treatment device 30 also comprises an optional absorption filter 48. The absorption filter 48 is arranged between the dichroic filter 34 and the light exit window 10 such that the transmitted light component 40 passes through the absorption filter 48. The absorption filter 48 can be a standalone optical component, or it can be embedded in an optical waveguide. The absorption filter 48 is configured to absorb wavelengths of light corresponding to the unwanted light, in case light having wavelengths below (in this example) the cut-off wavelength of the dichroic filter 34 finds its way to the light exit window 10. In particular, in practice the cut-off wavelength of a dichroic filter 34 is not typically a sharp cut-off, but has a transition across a finite wavelength width. In addition, the effectiveness of the dichroic filter 34 can be affected by the angle at which light is incident on the dichroic filter 34. Thus, some light having wavelengths below the cut-off wavelength of the dichroic filter 34 can be transmitted by the dichroic filter 34, and is absorbed by the absorption filter 48. With good design of the dichroic filter, i.e. incorporating sufficient dielectric layers, the leakage of unwanted light through the dichroic filter 34 can be limited so that the power absorbed in the absorption filter 48 is a small fraction of the power absorbed by an absorption filter in a conventional absorption filter-only design. Effectively, the reflected light component is removed from the light engine of the treatment device 30, so that the recycling/reflection problem in conventional devices with a dichroic filter is avoided.

After the absorption filter 48 in the optical path of the transmitted light component 40 is an optional light guide 50 through which the filtered-transmitted light component 40 passes to exit the treatment device 30. The light guide 50 may be the light exit window 10, or may be part of the light exit window 10, or may be separate from the light exit window 10. In some embodiments, the light guide 50 is not present. In some embodiments, the light guide 50 may be part of a detachable attachment that is attached to the treatment device 30 to change the characteristics of the emitted light. For example, one type of attachment can provide a narrow aperture to reduce the area of skin exposed to the transmitted light component 40, whereas another type of attachment can provide a wider aperture to allow a larger area of skin to be exposed to the transmitted light component 40.

In Fig. 2, an optional gap 52 is shown between the beam dump 44 and a surface 54 of the prism 38 through which the reflected light component 42 exits the prism 38. This gap 52 can be an air gap, or a gap filled with another material that has a lower refractive index than the prism 38. This low refractive index gap or boundary 52 is beneficial to encourage or cause total internal reflection (TIR) of light from the light source 12 that is incident on the surface 54 before it encounters the dichroic filter 34. This gap 52 therefore avoids or minimises light components of the desired wavelengths (i.e. those that are to be part of the transmitted light component 40) being lost to the beam dump 44.

A further gap 56 is shown between the second prism 39 and absorption filter 48. This gap 56 is not essential, and the absorption filter 48 may be in contact with the second prism 39.

It will be noted that the low refractive index at the entrance side of the first prism 38 (which can be provided by the air in the cavity housing 32) can help to confine the reflected light component 42 (which will have a certain angular spread around the average propagation direction indicated by the arrow) to the prism 38 through total internal reflection before it exits the prism 38 via surface 54.

Turning now to Fig. 3, as noted above, in the set of embodiments represented by Fig. 3, the dichroic filter is such that the reflected light component is the component of the light that is to perform the treatment operation, and the reflected light component exits the treatment device via the light exit window. The transmitted light component is incident on, and is received by, the beam dump.

Thus, in Fig. 3 part of a treatment device 60 according to a second set of embodiments is shown. The treatment device 60 comprises a light source 12 arranged in a cavity housing 62 of the treatment device 60. The light source 12 can be controllable or configured to generate pulses of light (e.g. IPL), or continuous, or relatively continuous, light.

The cavity housing 62 forms a reflective cavity for the light source 12 so that generated light is directed generally towards a dichroic filter 64, as indicated by arrow 66. The dichroic filter 64 is part of a prism 68. The dichroic filter 64 may be a thin-film dichroic filter. The prism 68 is located at the exit of the reflective cavity formed by the cavity housing 62. The dichroic filter 64 may be formed or provided on one of the surfaces of the prism 68, in which case a second prism 69 is provided that abuts the surface with the dichroic filter 64 in the prism 68. Alternatively, the dichroic filter may be formed on one of the surfaces of prism 69, in which case the first prism 68 abuts the surface with the dichroic filter in prism 69. Light transmitted through the dichroic filter 64 passes through the second prism 69. Alternatively, the dichroic filter 64 may be provided on an internal surface of a generally cubic or cuboid prism (and so comprising prism elements 68 and 69 in Fig. 3). In this configuration, in which the transmitted light component 72 is incident on, and is received by, the beam dump 74, the second prism 69 may be thin plate (cuboid prism) in case the beam dump 74 is in optical contact with it, or the second prism 69 can be omitted in which case the beam dump is in direct optical contact with the dichroic filter 64.

The prism 68 can be a solid prism, in which case the second prism 69 may also be a solid prism.

The dichroic filter 64 is arranged at a first angle with respect to the incident light (arrow 66), and acts to filter the incident light 66 by transmitting some of the light in the incident light 66 through the dichroic filter 64 and reflecting some of the light in the incident light 66 from the dichroic filter 64 according to a cut-off wavelength of the dichroic filter.

In the embodiment illustrated in Fig. 3, the light that is to be the treatment light is light having wavelengths above the cut-off wavelength, and thus the dichroic filter 64 is configured to reflect light having wavelengths above the cut-off wavelength, with light having wavelengths below the cut-off wavelength being transmitted through the dichroic filter 64. The reflected light (the treatment light, in this embodiment) is indicated by arrow 70, and the transmitted light (the unwanted light, in this embodiment) is indicated by arrow 72. The cut-off wavelength can be between 500 nm and 600 nm, e.g. 530 nm or 565 nm. If the type of treatment is other than hair removal/reduction, the cut-off wavelength may have a different value.

The transmitted light 70 is directed towards the light exit window 10, through which it can exit the treatment device 60. The transmitted light 72 is directed towards a beam dump 74 that absorbs light, and in particular absorbs light having wavelengths at least in the range corresponding to the wavelengths of light in the transmitted light 72. Coupled to the beam dump 74 is a heat sink 76 that acts to dissipate heat from the beam dump 74.

In this illustrated embodiment, the dichroic filter 64 is arranged at an angle of 45° with respect to the incident light 66, with the light exit window 10 arranged at an angle of 90° with respect to the incident light 66. However, in alternative embodiments, the angle of the dichroic filter 64 with respect to the incident light 66 can be other than 45°. For example, the angle may be close to 45°, or any angle between 30° and 60°. Likewise, the angle of the light exit window 10 with respect to the incident light 66 may be other than 90°, although preferably the light exit window 10 is positioned with respect dichroic filter 64 to receive as much of the reflected light component 70 as possible.

As the beam dump 74 and heat sink 76 are positioned away from the light exit window 10, which is typically placed into contact with the skin of the subject, the treatment device 60 will not feel as hot to the subject during use.

The beam dump 74 can be formed from any suitable material and have any suitable construction. For example, the beam dump can be a conical beam trap of a blackened material, such as a metal. More simply, the beam dump 74 can be a "black" layer with high absorption and low reflectance. Specific examples of suitable black materials are black anodized aluminium, nickel-phosphorus alloy and coatings based on carbon nanotubes.

The heat sink 76 can be formed from any suitable material and have any suitable construction. For example, the heat sink can be a base with fins to increase the contact area with an air flow through the fins. It can be made out of a single piece of material like copper or an aluminium alloy. Alternatively, sheet-metal fins can be soldered onto the base.

As noted above, in the embodiment of Fig. 3, in addition to the dichroic filter 64, the treatment device 60 also comprises an optional absorption filter 78. The absorption filter 78 is arranged between the dichroic filter 64 and the light exit window 10 such that the reflected light component 70 passes through the absorption filter 78. The absorption filter 78 can be a standalone optical component, or it can be embedded in an optical waveguide. The absorption filter 78 is configured to absorb wavelengths of light corresponding to the transmitted unwanted light, in case light having wavelengths below (in this example) the cut-off wavelength of the dichroic filter 64 finds its way to the light exit window 10. In particular, in practice the cut-off wavelength of a dichroic filter 64 is not typically a sharp cut-off, but has a transition across a finite wavelength width. In addition, the effectiveness of the dichroic filter 64 can be affected by the angle at which light is incident on the dichroic filter 64. Thus, some light having wavelengths below the cut-off wavelength of the dichroic filter 64 can be reflected by the dichroic filter 64, and is absorbed by the absorption filter 78. With good design of the dichroic filter, i.e. incorporating sufficient dielectric layers, the leakage of unwanted light through the dichroic filter 64 can be limited so that the power absorbed in the absorption filter 78 is a small fraction of the power absorbed by an absorption filter in a conventional absorption filter-only design. Effectively, the transmitted light component is removed from the light engine of the treatment device 60, so that the recycling/reflection problem in conventional devices with a dichroic filter is avoided.

After the absorption filter 78 in the optical path of the reflected light component 70 is an optional light guide 80 through which the filtered-reflected light component 70 passes to exit the treatment device 60. The light guide 80 may be the light exit window 10, or may be part of the light exit window 10, or may be separate from the light exit window 10. In some embodiments, the light guide 80 is not present. In some embodiments, the light guide 80 may be part of a detachable attachment that is attached to the treatment device 60 to change the characteristics of the emitted light. For example, one type of attachment can provide a narrow aperture to reduce the area of skin exposed to the reflected light component 70, whereas another type of attachment can provide a wider aperture to allow a larger area of skin to be exposed to the reflected light component 70.

In Fig. 3, an optional gap 82 is shown between the absorption filter 78 (or optical waveguide 80, if present, or otherwise simply the light exit window 10) and a surface 84 of the prism 68 through which the reflected light component 72 exits the prism 68. This gap 82 can be an air gap, or a gap filled with another material that has a lower refractive index than the prism 68. This low refractive index gap or boundary 82 is beneficial to encourage or cause total internal reflection (TIR) of light from the light source 12 that is incident on the surface 84 before it encounters the dichroic filter 64. This gap 82 therefore avoids or minimises light components of the unwanted wavelengths (i.e. those that are not to be part of the reflected light component 70) exiting the treatment device 60 via the light exit window 10 and being incident on the subject. Note that a hollow waveguide 80 adjacent to surface 84 effectively forms a low index boundary even if the waveguide is in contact with the prism 68.

In Fig. 3, another optional gap 86 is shown between the cavity housing 62 and the prism 68 into which the light 66 generated by the light source 12 enters. This gap 86 can be an air gap, or a gap filled with another material that has a lower refractive index than the prism 68. This low refractive index gap or boundary 86 is beneficial to encourage or cause total internal reflection (TIR) of a part of the reflected light component 70 (which will have a certain angular spread around the average propagation direction indicated by the arrow) that is incident on surface 90 before it leaves the prism 68 through surface 84. This gap 86 therefore avoids or minimises light intended for treatment being reflected back on the light source 12. Note that a hollow cavity housing adjacent to surface 90 effectively forms a low refractive index boundary even if the cavity housing is in contact with the prism 68.

The beam dump 74/heat sink 76 may be in contact with the surface of the second prism 69 through which the transmitted light component 72 exits the prism 69, or it may be spaced from the surface of the second prism 69 by a gap 88.

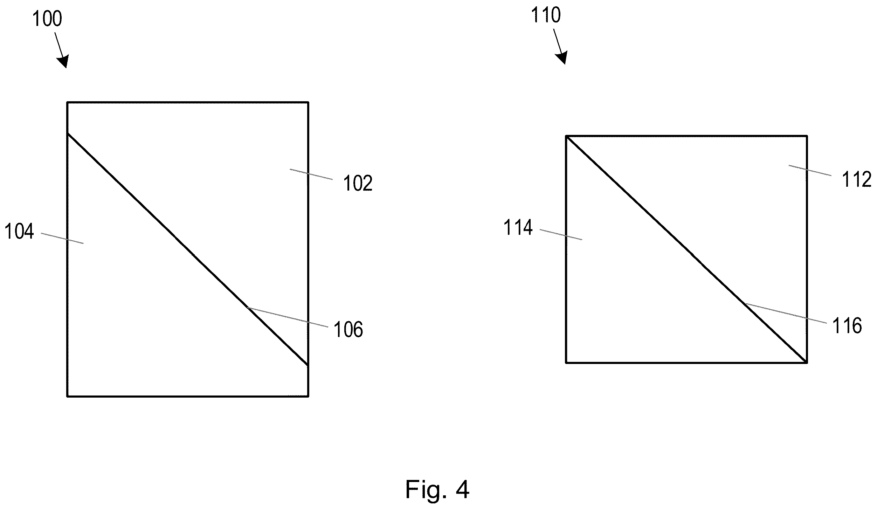

Figs. 4(a) and (b) show some exemplary dichroic filter and prism configurations. These examples can be used in the embodiments shown in Figs. 2 and 3. As noted above, the dichroic filter 34; 64 is part of a prism 38; 68. The dichroic filter 34; 64 may be formed on one of the surfaces of the prism 38; 68, in which case a second prism 39; 69 is provided that abuts the surface with the dichroic filter 34; 64 in the first prism 38; 68. Alternatively, the dichroic filter 34 may be an internal surface of a generally cubic or cuboid prism (and so comprising prism elements 38 and 39 in Fig. 2, or elements 68 and 69 in Fig. 3).

A prism with embedded or integral dichroic filter can be made in a similar way as a dichroic cube or cuboid. It starts with two complementary prisms, e.g. prisms 38 and 39 or 68 and 69. These prisms can be made of glass or an optical polymer. A dielectric multi-layer filter is deposited on one of the prisms, on the surface that will be in contact with the other prism. Typically, this multi-layer dichroic filter is symmetrical so that the dichroic filter function is identical for both directions of incidence. The two prisms can be bonded together by an adhesive or cement, or by optical contacting. Fig. 4(a) shows a rectangular cuboid prism 100 with a dichroic filter 106 embedded between a first solid prism 102 and a second solid prism 104. The prisms 102, 104 are truncated cuboids. Fig. 4(b) shows an alternative configuration in the form of a dichroic cube 110 made from two right prisms 112, 114 with right isosceles triangular end faces, and the dichroic filter 116 between them.

In some embodiments, one or more surfaces in the arrangements shown in Figs. 2 and 3 may be provided with an anti-reflective coating to minimise the recycling/reflection of the unwanted light and/or maximise transmission of treatment light. For example, surfaces where the unwanted light component exits a prism or other solid light guide into a medium with a lower refractive index (typically air). Otherwise, Fresnel reflections at the interface reflect some energy in the unwanted wavelength range back into the optical system, which increases the heat load on the absorption filter 48, 78 (if present), or increases the amount of unwanted light incident on the subject via the light exit window 10. In the arrangement shown in Fig. 2, an anti-reflective coating can be applied to surface 54. In the arrangement shown in Fig. 3, an anti-reflective coating can be applied to the surface adjacent to the beam dump 74 through which the transmitted light component 72 is to exit the second prism 69, if the beam dump 74 is not in optical contact with this exit surface. As another example, surfaces where the treatment light exits a prism into a medium with lower refractive index may be provided with an anti-reflection coating. Otherwise, Fresnel reflections at the interface reflect some energy in the treatment wavelength range and thus reduce the amount of treatment light reaching the skin. In the arrangement shown in Fig. 2, an anti-reflective coating can be applied to the surface where the transmitted component 40 exits the prism 39. In the arrangement shown in Fig. 3, an anti-reflective coating may be applied to surface 84. For the same reason, it can be useful to apply an anti-reflection coating on surfaces where the light generated by the light source enters a prism from a medium with a lower refractive index.

Simulation results for a multi-layer dichroic filter with a cut-off wavelength of around 600 nm have shown that a large percentage of the incident light power below the cut-off wavelength of 600nm would be transmitted to the beam dump 74 and heat sink 76. The results also show that the higher wavelengths (i.e. above 600nm) are reflected by the dichroic filter 64 towards the light exit window 10 and the skin of the subject.

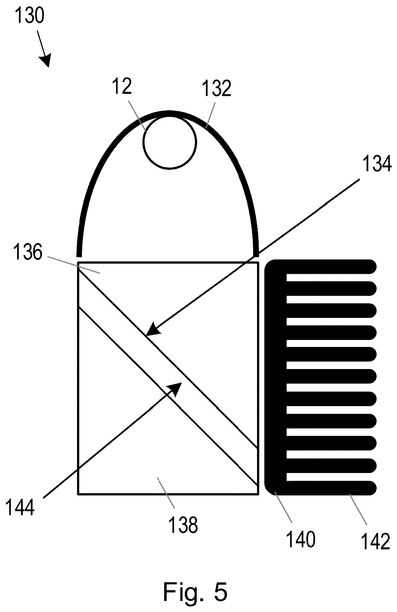

Fig. 5 is a simplified illustration of part of a treatment device 130 according to a third set of embodiments. In the third set of embodiments, the treatment device 130 comprises a light source 12, cavity housing 132, dichroic filter 134, prism 136, second prism 138, beam dump 140, and heat sink 142, that can correspond to, and/or be configured and/or used in the same way as the light source 12, cavity housing 32, dichroic filter 34, prism 38, second prism 39, beam dump 44, and heat sink 46 respectively in the first set of embodiments described above with reference to Fig. 2.

In the third set of embodiments, the treatment device 130 comprises a transmissive absorption filter 144 that is combined with the dichroic filter 134. For example, the dichroic filter 134 can be deposited on top of an absorption filter plate 144. The absorption filter 144 is configured to pass the light component in the treatment wavelength range, and absorb the wavelengths of light in the unwanted wavelength range. In this way, the number of interfaces for the light is reduced so that reflective light losses can be minimised, while the reduced absorption of light by the absorption filter 144 (due to the majority of the unwanted light being reflected by the dichroic filter 134) means that the temperatures are maintained low enough such that all components (absorption filter 144 (with dichroic filter 134) and prism 136 and 138) can be glued or optically contacted.

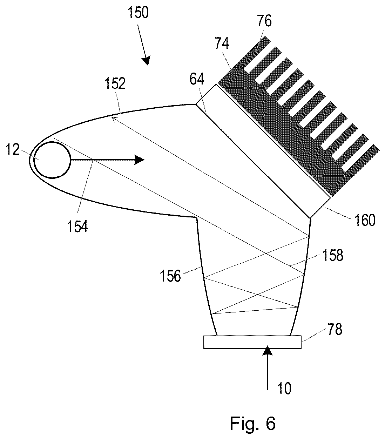

As noted above, the presented embodiments can reduce the amount of unwanted light reaching the absorption filter (if present) or exiting the treatment device via the light exit window. The fourth set of embodiments discussed below with reference to Fig. 6 is a variant of the second configuration, wherein the dichroic filter is such that the reflected light component is the component of the light that is to perform the treatment operation, and the reflected light component exits the treatment device via the light exit window. This variant provides an alternative to the use of a solid prism 68.

Briefly, the fourth set of embodiments expand on the second set of embodiments shown in Fig. 3, and provide a reflective cavity design to promote that direct emission of light from the light source towards the light exit window (i.e. any light that has not been incident on the dichroic filter) is prevented, ensuring that the light is efficiently filtered.

Thus, Fig. 6 is an illustration of part of a treatment device 150 according to a fourth set of embodiments. Components and features of the treatment device 150 that are common to the treatment device 60 according to the second set of embodiments in Fig. 3 use the same reference numerals, and the details provided above in respect of those components and features apply also to treatment device 150. Thus, the cavity housing 152 forms a reflective cavity for the light source 12 so that generated light is directed generally towards the dichroic filter 64, as indicated by arrow 154. This cavity housing 152 is referred to as a diverging cavity section 152, as it shapes/reflects light generated by the light source towards the dichroic filter 64, so that the angular distribution of the light emerging from the diverging cavity section 152 is concentrated around the optical axis 154. In some embodiments, the diverging cavity section 152 is or resembles a conic section.

Despite the angular distribution of the light being narrowed via the diverging cavity section 152, it is still possible for light to avoid the dichroic filter 64 and be directed towards the light exit window 10. Therefore, a converging cavity section 156 is provided before the light exit window 10. In some embodiments, the light exit window 10 can be at or near the apex of the converging cavity section 156. As shown in Fig. 6, the diverging cavity section 152 and the converging cavity section 156 are angled 90° (or close to 90°) with respect to each other. In some embodiments, the converging cavity section 156 is or resembles a conic section.

The converging cavity section 156 is shaped and positioned to reflect light entering the converging cavity section 156 directly from the diverging cavity section 152 (i.e. light that has not interacted with the dichroic filter 64 first) back towards the diverging cavity section 152 (possibly via the dichroic filter). This reflecting light is represented by arrows 158. Therefore, if light 154 from the light source 12 directly enters the converging cavity section 156, it is directed back towards the light source 12 via reflections from the walls of the converging cavity section 156 before the light can reach the light exit window 10. Consequently, direct emission from the light source 12 towards the light exit window 10 is prevented, ensuring that essentially all the light is efficiently filtered by the dichroic filter 64, except potentially a small (< a few percent) fraction (depending on the exact design of the diverging and converging sections) and/or light that is scattered from residual surface roughness in the reflectors.

In the embodiment of Fig. 6, the dichroic filter 64 is a surface of a prism 160, which can be rectangular or generally rectangular in cross-section. The beam dump 74 and heat sink 76 are arranged adjacent to an opposite surface of the prism 160, so that light transmitted by the dichroic filter 64 passes through the prism 160 and into the beam dump 74.

The beam dump 74/heat sink 76 may be in contact with the surface of the prism through which the transmitted light component exits the prism, or it may be spaced from the surface of the prism 160.

In some embodiments, an absorption filter 78 can additionally be provided to absorb any potentially harmful or unwanted parts of the spectrum of this residual light and/or light not perfectly filtered by the dichroic filter from reaching the skin. Since the amount of light needing to be absorbed by the absorption filter 78 is strongly reduced, as in previous configurations, there can be little temperature increase of the absorption filter 78 during operation of the treatment device 150. The absorption filter 78 can also act as an electric isolator for the high voltages on the diverging cavity section 152, while also acting as the light exit window 10 for efficient light delivery and IPL pain mitigation.

Simulations have shown that, with careful design of the diverging and converging cavity sections 152, 156 the undesired part of the unfiltered exposure dose on an absorption filter at or near the light exit window 10 can be reduced to only a few percent of the total, allowing this absorption filter to remain essentially at room temperature and safe to the touch under all treatment conditions.

Therefore there is provided a treatment device in which the unwanted light component is directed to the beam dump where it is absorbed, and the heat generated in the beam dump by the absorption of light is dissipated via the coupled heat sink. This reduces the build-up of heat in the treatment device, and thereby improves the user experience when handling the treatment device.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A treatment device (2; 30; 60; 130) for performing a light-based treatment operation on or to a subject, the treatment device (2; 30; 60; 130) comprising:
a light source (12) for generating light for performing the treatment operation;
a dichroic filter (34; 64; 104; 134) arranged at a first angle with respect to incident light such that the incident light is separated into a transmitted light component and a reflected light component according to a cut-off wavelength of the dichroic filter (34; 64; 104; 134), wherein the transmitted light component is transmitted through the dichroic filter (34; 64; 104; 134) and the reflected light component is reflected by the dichroic filter (34; 64; 104; 134);
a light exit window (10) arranged with respect to the dichroic filter (34; 64; 104; 134) such that one of the transmitted light component and the reflected light component is emitted from the treatment device (2; 30; 60; 130) via the light exit window (10);
a beam dump (44; 74; 140) configured and arranged with respect to the dichroic filter (34; 64; 104; 134) such that the other one of the transmitted light component and reflected light component is incident on the beam dump (44; 74; 140) and absorbed by the beam dump (44; 74; 140); and
a heat sink (46; 76; 142) coupled to the beam dump (44; 74; 140) to dissipate heat from the beam dump (44; 74; 140);
**characterized in that** the treatment device comprises a transmissive absorption filter (144) configured to pass the light component in a treatment wavelength range, and absorb the wavelengths of light in an unwanted wavelength range,
wherein the dichroic filter (134) is deposited on top of the transmissive absorption filter (144).

2. The treatment device (2; 30; 60; 130) of claim 1, wherein the absorption filter (48; 78; 144) is configured to absorb light having wavelengths corresponding to the wavelengths of the other one of the transmitted light component and the reflected light component.

3. The treatment device (2; 30; 60; 130) of any of claims 1-2, wherein the dichroic filter (34; 64; 104; 134) is provided on a first surface of a solid dichroic prism (38; 68), wherein a second prism (39; 69) is arranged in contact with the first surface of the solid dichroic prism (38; 68) such that the light component transmitted by the dichroic filter (34; 64; 104; 134) passes through the second prism (39; 69).

4. The treatment device (2; 30; 60; 130) of claim 3, wherein a low refractive index boundary is provided at a second surface of the dichroic prism (38; 68) through which the reflected light component is to exit the dichroic prism (38; 68), wherein the low refractive index boundary has a lower refractive index than the dichroic prism (38; 68).

5. The treatment device (2; 30; 60; 130) of any of claims 1-2, wherein the dichroic filter (34; 64; 104; 134) is provided on an internal surface in a solid dichroic cuboid, wherein a low refractive index boundary is provided at a first surface of the dichroic cuboid through which the reflected light component is to exit the dichroic cuboid, wherein the low refractive index boundary has a lower refractive index than the dichroic cuboid.

6. The treatment device (2; 30; 60; 130) of any of claims 1-5, wherein the dichroic filter (34; 64; 104; 134), light exit window (10) and beam dump (44; 74; 140) are configured with respect to each other such that the transmitted light component is emitted from the treatment device (2; 30; 60; 130) via the light exit window (10) and the reflected light component is incident on the beam dump (44; 74; 140).

7. The treatment device (2; 30; 60; 130) of claim 6, wherein the dichroic filter (34; 64; 104; 134) is configured such that the transmitted light component comprises light having wavelengths above the cut-off wavelength, and the reflected light component comprises light having wavelengths below the cut-off wavelength.

8. The treatment device (2; 30; 60; 130) of any of claims 1-5, wherein the dichroic filter (34; 64; 104; 134), light exit window (10) and beam dump (44; 74; 140) are configured with respect to each other such that the reflected light component is emitted from the treatment device (2; 30; 60; 130) via the light exit window (10) and the transmitted light component is incident on the beam dump (44; 74; 140).

9. The treatment device (2; 30; 60; 130) of claim 8, wherein the dichroic filter (34; 64; 104; 134) is configured such that the transmitted light component comprises light having wavelengths below the cut-off wavelength, and the reflected light component comprises light having wavelengths above the cut-off wavelength.

10. The treatment device (2; 30; 60; 130) of claim 8 or 9, wherein the treatment device (2; 30; 60; 130) comprises a diverging cavity section (152) and a converging cavity section (156), wherein the light source (12) is arranged towards a first end of the diverging cavity section (152), the light exit window (10) is arranged at an end of the converging cavity section (156), and the dichroic filter (34; 64; 104; 134) is arranged between the diverging cavity section (152) and the converging cavity section (156).

11. The treatment device (2; 30; 60; 130) of claim 10, wherein the converging cavity section (156) and diverging cavity section (152) are configured such that light entering the converging cavity section (156) without reflecting from the dichroic filter (34; 64; 104; 134) is reflected by the converging cavity section (156) away from the light exit window (10) and towards the diverging cavity section (152).
